# EUROPEAN PATENT SPECIFICATION

(11) **EP 2 150 193 B1**
(45) Date of publication and mention of the grant of the patent: **03.06.2015**
(21) Application number: 08745928.5
(22) Date of filing: 16.04.2008
(51) Int. Cl.: A61B 18/14, A61B 17/00, A61B 18/00

(54) **ABLATION CATHETER WITH FLEXIBLE TIP**
ABLATIONSKATHETER MIT FLEXIBLER SPITZE
CATHETER D'ABLATION A EMBOUT FLEXIBLE

(30) Priority: 23.05.2007 US 939799 P; 11.09.2007 US 853759
(43) Date of publication of application: 10.02.2010
(73) Proprietor: Irvine Biomedical, Inc., Irvine, CA 92614 (US)
(72) Inventor: PAPPONE, Carlo, 23870 Lecco (IT); DE LA RAMA, Alan, Cerritos, California 90703 (US); CHEN, Peter, Cheng, Irvine, California 92603 (US); HATA, Cary, Irvine, California 92620 (US); SHIMIZU, Jared, A., Carmichael, California 95608 (US)
(74) Representative: Kramer Barske Schmidtchen Patentanwälte PartG mbB
(86) International application number: PCT/US2008/060420
(87) International publication number: WO 2008/147599

(56) References cited:
- WO-A1-03/049631
- US-A- 5 681 280
- US-A- 5 951 471
- US-A- 6 001 095
- US-A1- 2001 012 956
- US-A1- 2003 088 244
- US-A1- 2004 015 215
- US-A1- 2004 153 056
- US-A1- 2004 181 138
- US-A1- 2004 243 143
- US-A1- 2005 004 563
- US-A1- 2006 064 123
- US-B1- 6 464 632

## Description

### BACKGROUND OF THE INVENTION

This invention relates generally to catheter devices, and more specifically to a flexible electrode structure for catheter tips.

Catheters are flexible, tubular devices that are widely used by physicians performing medical procedures to gain access into interior regions of the body. Catheters with one or more electrodes are generally known in the surgical art. Electrode catheters can be used for electrically mapping a body part, to deliver therapy to a body part, or both.

For treating heart conditions such as arrhythmia, catheters having ablation electrodes are used to create lesions in heart tissues. Such lesions may be linear lesions or single point lesions. A single point lesion, as its name implies, is created by applying ablation energy at a single point region of tissue. On the other hand, linear lesions involve applying ablation energy over a larger area in an elongated region of tissue. Linear lesions are known to have some advantages over mere single point lesions.

Creating a linear lesion with only a conventional tip electrode, however, is relatively time-consuming, labor-intensive, and generally impractical. A surgeon may attempt to use a typical single point electrode catheter to create a linear lesion, by carefully dragging the single point electrode tip across the tissue while applying energy to the tissue surface, but this is difficult to accomplish successfully.

U.S. Patent No. 5,487,385 discloses a flexible catheter having ring electrodes disposed along its flexible shaft for creating a linear lesion. A surgeon may lay the catheter shaft with the electrodes across a tissue area, and allow the consecutively-arranged ring electrodes to ablate the target tissue using RF energy. The ring electrodes, however, must apply sufficient energy in order to create lesion areas that are connected, thus forming a single linear lesion. Applying too much RF energy, however, can cause unwanted tissue damage, and this arrangement often results in a series of spaced-apart single point lesions.

U.S. Patent No. 6,063,080 discloses a catheter having an elongated electrode. This electrode has micro-slotting or micro-apertures across its surface to improve flexibility of the electrode, thus allowing a surgeon to lay the elongated electrode across a tissue surface. Despite some desirable properties, such a longitudinal-type electrode has several disadvantages. For example, the electrode requires a spherical structure at its tip to prevent the electrode from penetrating tissue. Also, a longitudinal type electrode cannot effectively create a linear lesion when the electrode is laid across a tissue having ridges.

US 5681280 A and US 2004/153056 A1 disclose a basket electrode for a steerable catheter, respectively.

US 2004/181138 A1 discloses a steerable catheter having an irrigated electrode at its distal end. A hinge section is provided which allows the intermediate shaft to bend.

US 2003/088244 A1 discloses an ablation device comprising a curvilinear distal section with curvilinear electrode elements. The device comprises a rigid metal electrode at its tip and another rigid electrode which is provided more proximally. The body in its entirety can be flexed between the electrodes.

US 2004/015215 A1 refers to a system and method for approaching the intervertebral disc for thermally treating the intervertebral disc. The device uses radiofrequency electrodes. An electrode having gaps is provided.

US 6464632 B1 discloses an inner liner for an electrode device. The inner liner is provided with gaps.

US 2006/064123 A1 discloses a bendable polymer structure. This bendable polymer structure has gaps.

WO-A-03 049 631 discloses an irrigated electrosurgical catheter.

### BRIEF DESCRIPTION OF THE INVENTION

The invention provides an ablation catheter according to claim 1. Preferred embodiments are defined in the dependent claims.

### BRIEF DESCRIPTION OF THE DRAWINGS

Figure 1 is a perspective view of an exemplary flexible tip electrode for a catheter not showing all features of the invention.
Figures 1A-1C illustrate alternative examples of the flexible tip electrode shown in Figure 1.
Figure 2 is a perspective view of another example of a flexible tip electrode for a catheter not showing all features of the invention.
Figures 2A-2D illustrate alternative examples of the flexible tip electrode shown in Figure 2.
Figure 3 is a perspective view of another example of a flexible tip electrode for a catheter not showing all features of the invention.
Figure 3A is a side view of the tip electrode shown in Figure 3.
Figure 4 is a perspective view of another example of a flexible tip electrode not showing all features of the invention.
Figures 4A-4B are alternative examples of the flexible tip electrode shown in Figure 4.
Figure 5 is a perspective view of another example of a flexible tip electrode for a catheter not showing all features of the invention.
Figure 6 is a perspective view of another example of a flexible tip electrode for a catheter not showing all features of the invention.
Figures 7A-7C illustrate embodiments of catheters having flexible electrode tips in use.
Figures 8A-8C illustrate further embodiments of catheters having flexible electrode tips in use.
Figure 8D is a cross-sectional view of a portion of Figure 8B taken along line A-A.
Figure 8E schematically illustrates an altered cross sectional shape for the electrode tip shown in Figure 8C.
Figure 9 is a side elevational view of a portion of an embodiment of a flexible tip electrode according to the invention.
Figure 9A is a magnified view of a portion of Figure 9.
Figure 10 is a side elevational view of a section of the electrode tip shown in Figure 9.
Figure 11 is a view of an alternative section of an electrode tip.
Figure 12A is schematic illustration of a section of a tip electrode.
Figure 12B is a side elevational view of a flexible tip electrode showing a degree of flexing.
Figure 12C is a side elevational view of the tip electrode shown in Figure 12B being dragged across tissue having ridges thereon.
Figure 12D is aside elevational view of the tip electrode being dragged across smooth tissue surface.
Figure 13 is a side elevational view of a portion of the tip electrode shown in Figure 9.
Figure 13A is a side view of the tip electrode depicted in Figure 13 at rest.
Figure 13B is a side view of the tip electrode in Figure 13 when pressed against a tissue surface.
Figure 14A is a longitudinal cross-sectional view of a further embodiment of a tip electrode.
Figure 14B is a longitudinal cross-sectional view of still another embodiment of a tip electrode.
Figure 15A is an illustrative view showing an exemplary electrode gap for forming a flexible tip electrode.
Figure 15B is an illustrative view of another electrode gap for forming a flexible electrode tip.

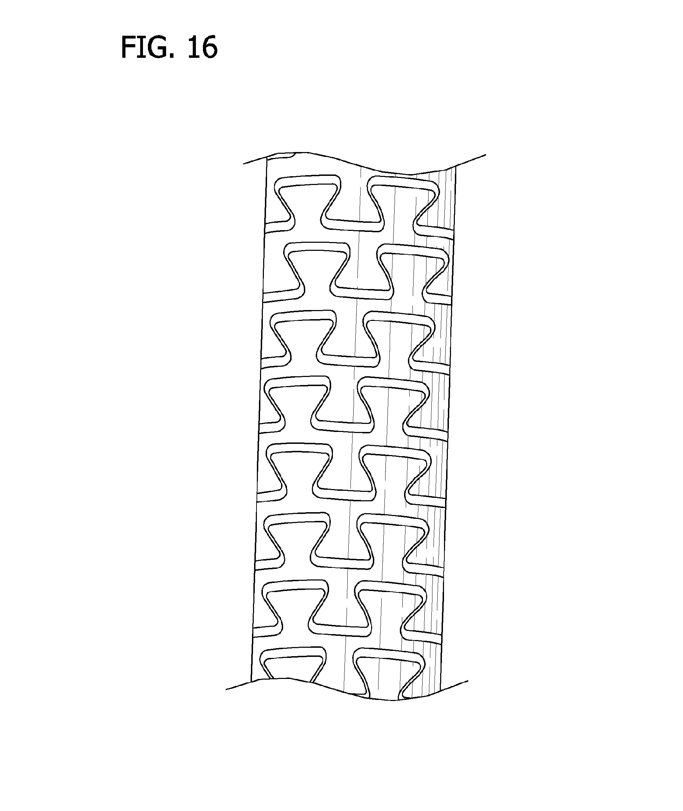

Figure 16 is a photograph of an exemplary tip electrode.

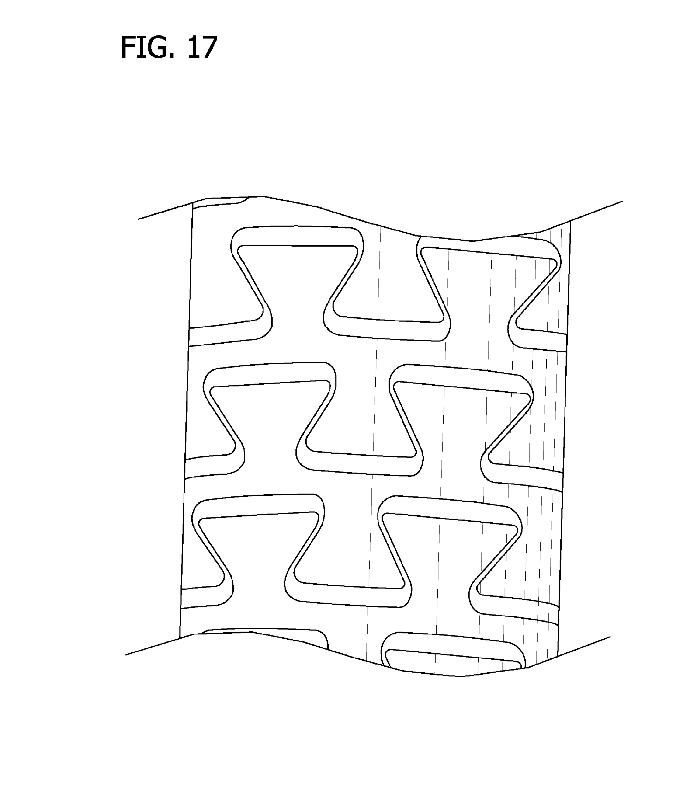

Figure 17 is a magnified view of a portion of the tip electrode shown in Figure 16.

### DETAILED DESCRIPTION OF THE INVENTION

Embodiments of ablation catheters having tips including flexible and bendable electrodes, and also freedom of movement to shorten an axial length of the catheter tip, while reliably creating linear lesions in body tissues are disclosed. The flexibility of the electrodes increases an electrode-to-tissue contact area, and in turn improves ablation of tissue. Especially in tissue where ridges are present, the flexible tip electrodes can be dragged across the ridges with improved continuous electrode-to-tissue contact.

These and other benefits are accomplished by providing a flexible tip electrode for an ablation catheter that includes a generally hollow cylindrical structure with an interior lumen. A round dome-shaped terminal end may be provided. The cylindrical wall of the electrode may have a variety of different types of elongated grooves or openings defining gaps in the cylindrical wall, imparting some flexibility to the electrode to more capably conform to and establish sufficient surface contact with body tissues that may have irregular surface area including ridges and the like, and tissues that may be vibrating or moving. The grooves, openings and associated elongated gaps may have various shapes, sizes and overall configurations as explained below in numerous exemplary embodiments.

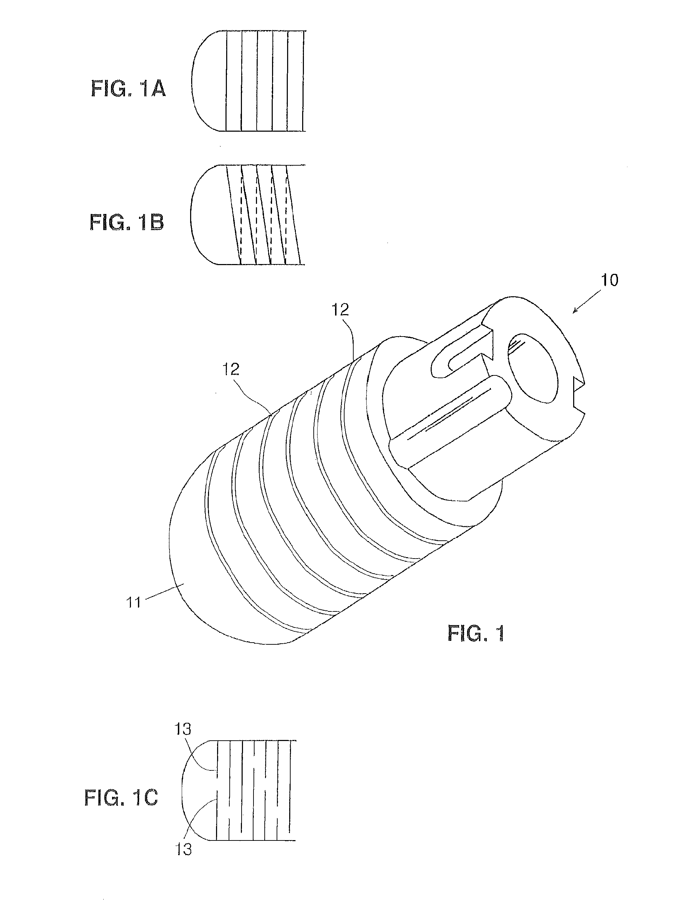

Referring now to Figure 1, an exemplary tip electrode 10 includes a hollow and generally cylindrical body having a dome tip 11 and a cylindrical sidewall. The sidewall may include a series of annular or ring-like surface channels or grooves 12 cut or otherwise formed into the sidewall. The grooves 12 define elongated areas of decreased wall thickness and decreased cross-sectional area of the sidewall, and hence the areas of the wall occupied by the elongated grooves 12 are structurally weaker and less rigid than areas of the sidewall where the grooves are not present, imparting flexible properties to the electrode wall. As used herein, an elongated groove preferably has a length that is at least about 3 times the width of the groove, more preferably at least about 5 times, and most preferably at least about 10 times.

As shown in Figure 1, the elongated grooves 12 are disposed about the tip electrode and extend generally parallel to one another. Each annular groove 12 extends in a plane that is generally perpendicular to a longitudinal axis of the tip. The respective grooves 12 may be spaced equidistant from each other along a longitudinal length of the tip electrode. Each annular groove 12 may form a continuous 360 degree unending loop (as illustrated in Figure 1A) that is circular. Alternatively, all or part of the series of ring grooves may extend in a non-circular and a non-planar helical configuration (as shown in Figure 1B) completing more than one 360 degree loop or turn on the surface of the electrode sidewall, with the helical ring grooves having discrete end points.

In another example, the electrode may include some annular rings extending in a plane that do not form a continuous unending loop, but rather grooves forming loops having two terminal ends 13 (as depicted in Figure 1C) that are spaced apart from one another. A further embodiment may include a combination of continuous and non-continuous, planar and non-planar groove configurations.

In alternative examples, elongated openings extending completely through the thickness of the sidewall of the electrode may be provided in lieu of elongated surface channels or grooves. As used herein, an elongated opening preferably has a length that is at least about 3 times the width of the opening, more preferably at least about 5 times, and most preferably at least about 10 times.

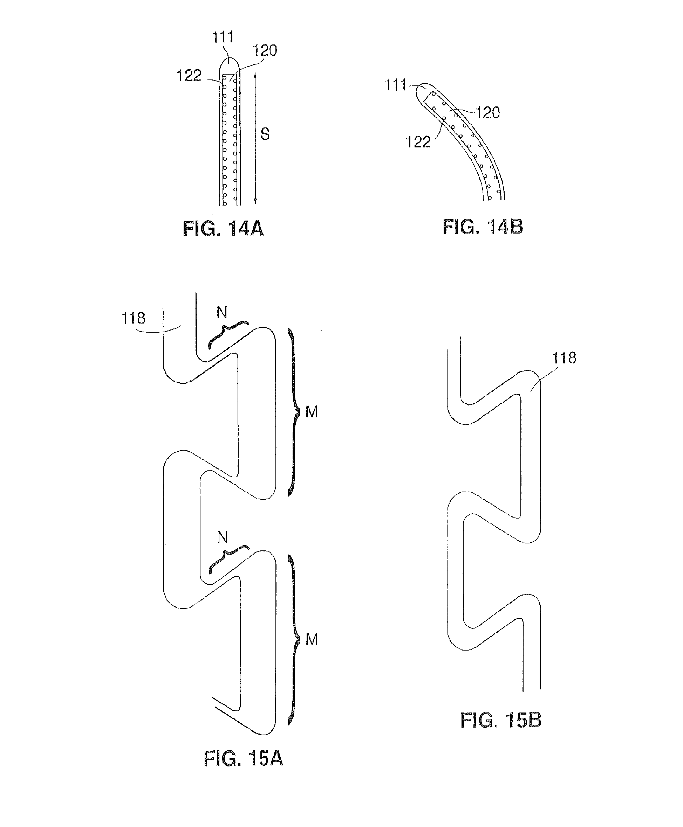

Elongated openings extending completely through the electrode sidewall will generally impart more flexibility, or less rigidity, in the sidewall than will elongated surface channel grooves. In the example shown in Figure 1A, however, if each complete loop is completely cut through the sidewall, some type of additional supporting structure is required to connect the severed-pieces together. For example, a biasing element such as an inner coil may be provided within the lumen, such as in the embodiments of Figures 14A and 14B to be described further below.

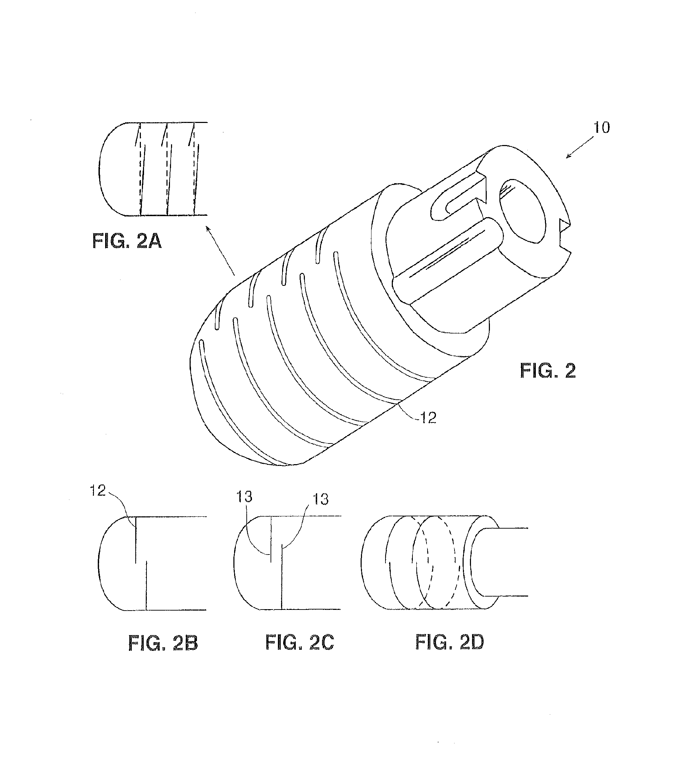

Referring now to Figure 2, the elongated ring-like, annular grooves 12 may be spaced further apart than in the example shown in Figure 1. Also, in the example of Figure 2, each ring-like, annular groove does not form a continuous, 360 degree loop on the electrode sidewall, and terminal ends of each respective groove are slightly offset or staggered relative to one another to maintain some degree of desired rigidity in the electrode sidewall. As shown in Figures 2A through 2C, the elongated grooves may have a circumferential length chosen so that the terminal ends 13 of adjacent grooves extend past one another for a specified distance in an interleaved or dovetail arrangement on the electrode sidewall. It is contemplated that in other examples a combination of continuous ringlike grooves, such as those shown in Figure 1, and non-continuous grooves such as those shown in Figure 2 may be utilized.

Figure 2D illustrates another example where offset elongated ring-like grooves 12 extend in respective planes spaced along a longitudinal axis of the tip, but the ring-like grooves extend only partly around the cylindrical sidewall of the electrode. In the example shown in Figure 2D, the elongated grooves 12 extend as staggered half loops extending across about 180 degrees of the cylindrical circumference of the electrode wall. Many other relative positions of half loops are also contemplated. In other embodiments, some or all the elongated grooves 12 may extend across more or less than 180 degrees of the cylindrical circumference of the electrode sidewall.

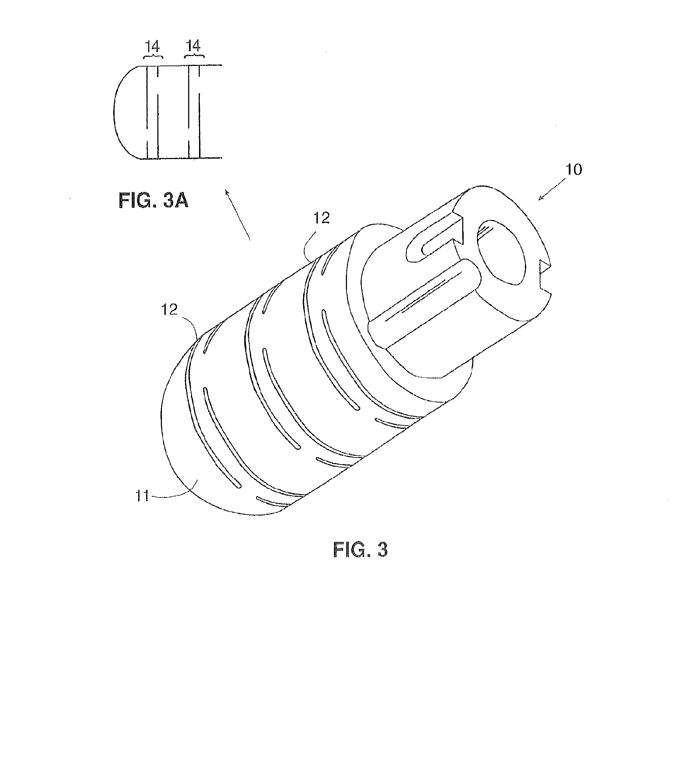

Figure 3 illustrates a tip electrode wherein three sets of elongated grooves 12 are provided, with each set 14 including two non-continuous ring-like loops. Spacing between the sets 14 is generally greater than spacing between the two ring-like loops in each set.

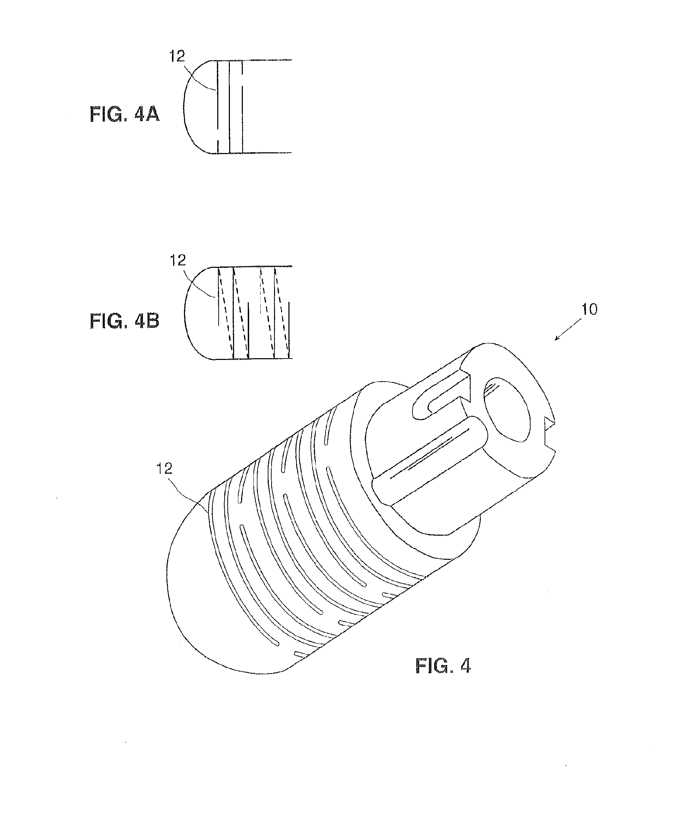

Figure 4 illustrates still further examples of a tip electrode. In the example shown in Figure 4A, the elongated ring-like grooves form non-continuous loops on the outer surface of the electrode sidewall, with each groove having terminal ends that do not connect with each other. In the example illustrated in Figure 4B, a series of elongated helical grooves 12 are provided that each extend for a number of turns on the surface of the electrode wall.

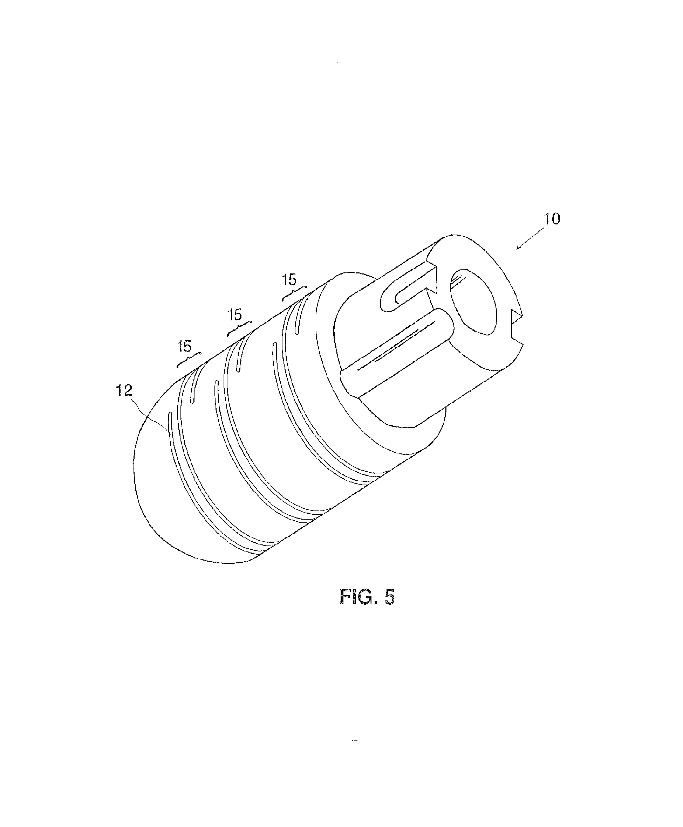

Figure 5 illustrates another catheter tip including three sets of elongated grooves 12. Each set 15 is shown to include a helical groove extending more than one 360 revolution or turn on the electrode sidewall.

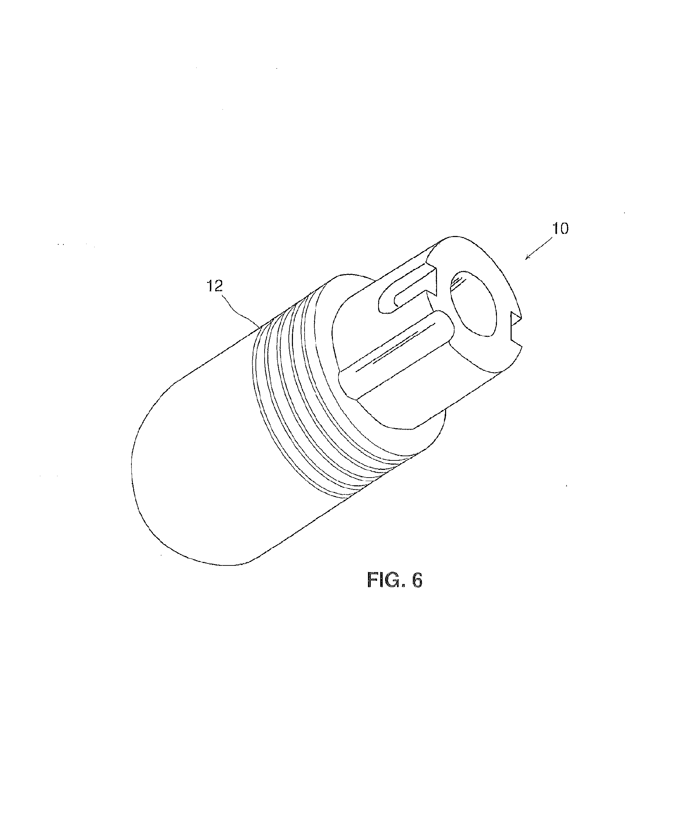

Figure 6 illustrates yet another contemplated example where a series of elongated, ring-like grooves 12 are disposed generally equidistant from each other only along a proximal section of the tip electrode 10. Each ring-like groove 12 may or may not form a continuous loop or a complete turn on the electrode sidewall. A combination of continuous loops and non-continuous loops are possible. By providing the elongated grooves 12 in the proximal end of the electrode tip, but not the distal end, a desired rigidity in the distal portion of the tip electrode 10 may be provided, while affording some flexibility to the proximal portion of the tip.

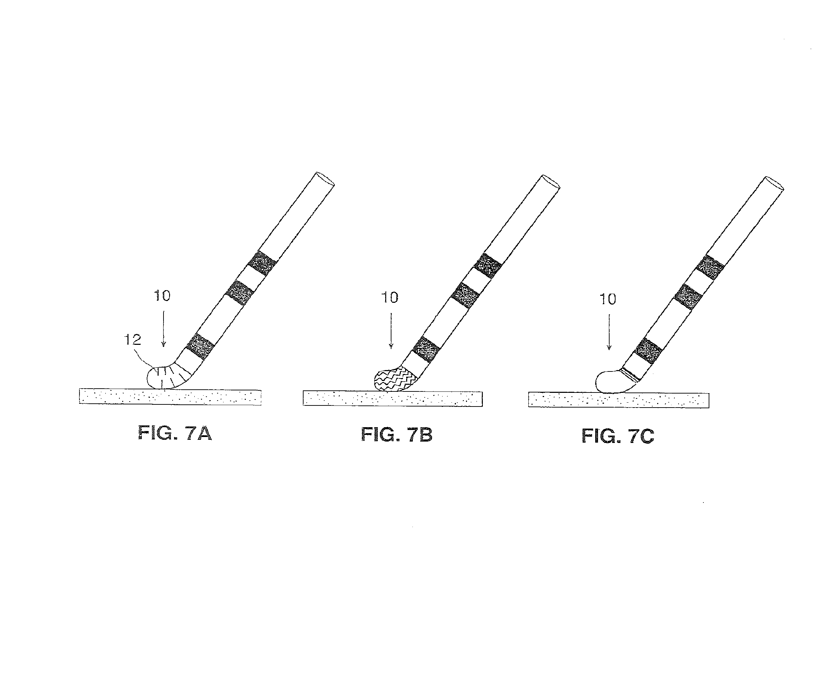

Figures 7A through 7C illustrate catheters including embodiments of flexible tip electrodes in use to map or ablate tissue surfaces. One aspect of the flexible tips allows and facilitates effective dragging of the flexible tip electrode 10 across a smooth tissue surface to create a linear lesion. This is possible because, as Figures 7A through 7C depict, the flexible tip electrode 10 may deform and/or flex when it is dragged across a tissue surface. The flexible and deformable properties of the flexible tips results in greater electrode-to-tissue surface area than would otherwise be possible with a rigid tip electrode.

In Figure 7A, the tip electrode includes an elongated cut pattern 12 that is similar to the example depicted in Figure 2D including relatively parallel rings or loops, but extending completely through the thickness of the electrode wall to impart additional flexibility in comparison to the grooved example of Figure 2D. In Figure 7B, a tip electrode with an elongated zig-zag cutting pattern allows even greater flexibility than the pattern of Figure 7A. In Figure 7C, however, the catheter tip electrode includes elongated grooves only at the neck region where the electrode is attached to the catheter (similar to the example of Figure 6), allowing some degree of flexibility in a more rigid tip electrode.

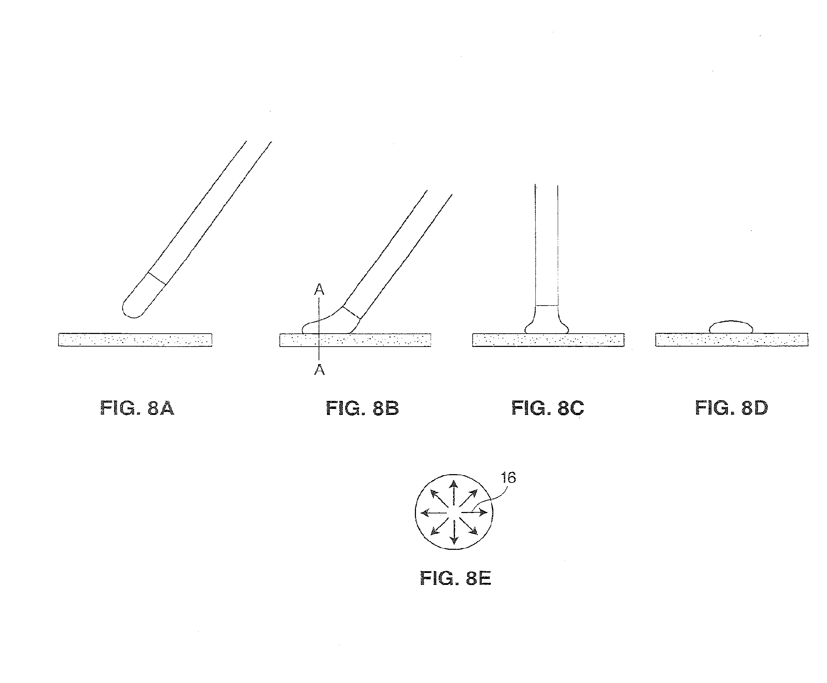

Figures 8A through 8E illustrate embodiments of the catheters having flexible tip electrodes that may advantageously deform, apart from simple bending along the longitudinal axis of the catheter body as shown in Figure 7, to create greater electrode-to-tissue surface area. In Figure 8A, a catheter including a flexible tip electrode is ready to make contact with a tissue surface. When the electrode makes contact with the tissue surface in Figure 8B, the tip electrode may deform. For example, a cross sectional area of the tip along line A-A in Figure 8B may become oval in shape as shown in Figure 8D. Such an electrode may not only flex along a longitudinal axis, but may also expand laterally when subjected to an applied force. When an angle between the tissue surface and a longitudinal axis of the catheter body gets closer to a 90 degree angle as shown in Figure 8C, the flexible tip may deform and shorten due to downward pressure against the tissue surface. Figure 8E, for example, schematically illustrates a cross section of the tip electrode in Figure 8C at a location parallel to the tissue surface. The electrode-to-tissue surface area, represented by the circle in Figure 8E is expandable outwardly in a direction of arrows 16 as the catheter is pressed further towards the tissue surface. One contemplated embodiment that has deformable properties as shown in Figures 8A through 8E is the tip electrode with an elongated zip-zag cut pattern as shown in Figure 7B.

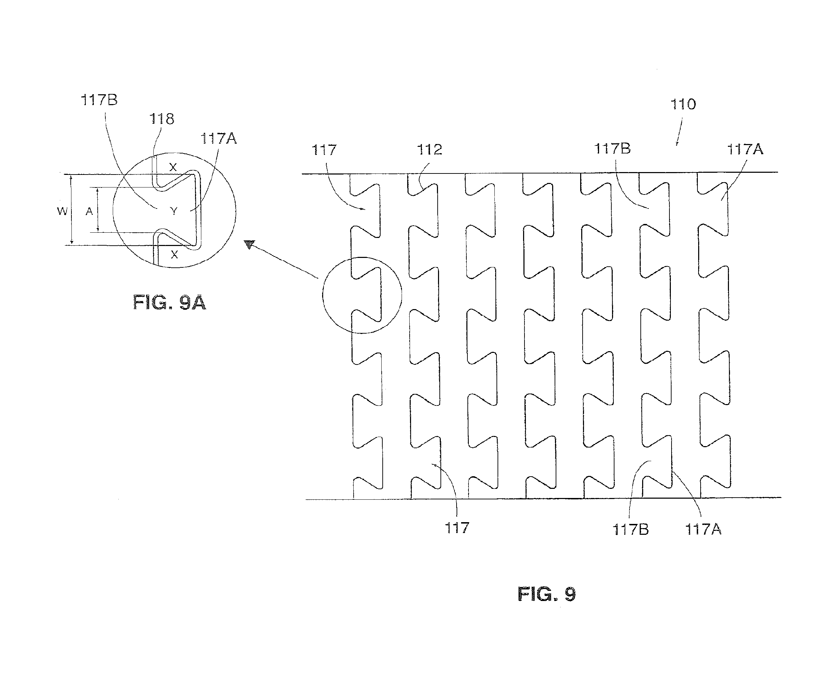

Referring now to Figure 9, an exemplary embodiment of flexible tip electrode 110 has an elongated cutting pattern in the electrode sidewall that outlines alternating interlocking blocks 117. In the illustrated embodiment, the contemplated blocks 117 are disposed on both sides of an elongated gap 118 created by the cutting pattern. Each block has a head 117A and a neck 117B, and the head 117A is wider than the neck 117B in each block. In the illustrated interlocking pattern, a first head, represented by "Y" in Figure 9A of the block 117, which has a neck 117B situated on one side of the gap 118, is disposed between a second and third heads represented by "X" in Figure 9A. The second and third heads X each have necks situated on the other side of the elongated gap 118 and opposing the head Y. The blocks X and Y are interlocked because the wider head portion of one block 117 is locked between the narrower neck portions of the two adjacent blocks 117. For example, the second and third heads X in Figure 9A are separated by a shortest distance A in Figure 9A, and distance A is shorter than a width W of the head Y, thereby restricting relative movement of two adjacent loops away from each other and preventing the blocks from separating.

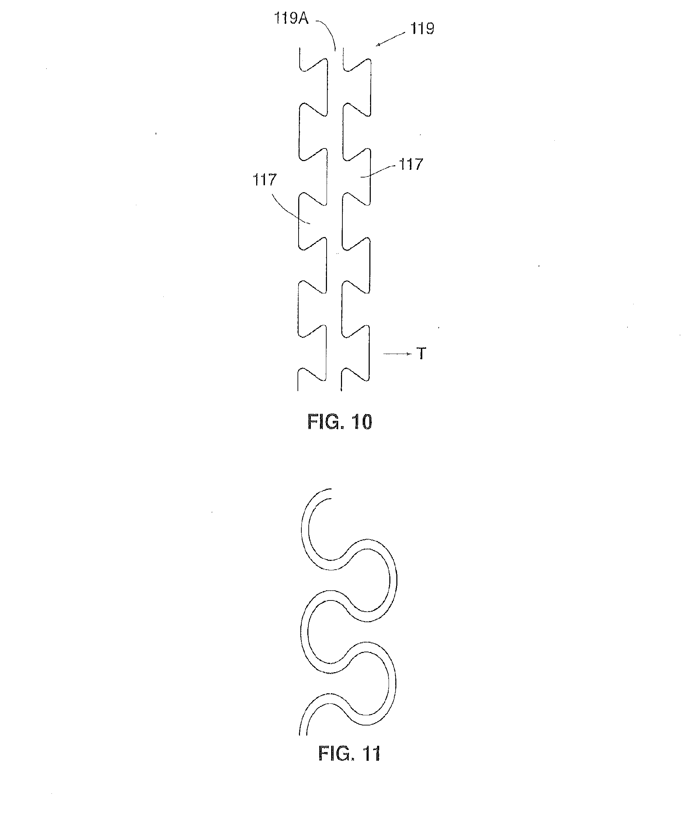

Contemplated patterns of elongated openings can also be described by focusing on the structures of the electrode wall, instead of focusing on the shape of the gap 118. For example, in Figure 10, a contemplated electrode wall includes a stem member 119 that may helically extend about a longitudinal axis of the electrode forming a series of stem loops (see Figure 9), and wherein the member 119 includes a plurality of protruding blocks 117 peripherally disposed on both sides of the stem member 119. Each block 117 transversely extends in a lateral direction indicated by arrow T in Figure 10 toward an adjacent stem loop in the electrode wall shown in Figure 9. Each adjacent stem loop includes blocks 117 that are staggered from the blocks 117 in immediately adjacent stem loops, resulting in an interlocking block pattern. Contemplated blocks for the stem member can have various shapes. For example, at least some of the blocks 117 may have a shape of an upside down triangle as illustrated, where one angle of the triangle represents the neck region. Alternatively, blocks with rounded bulbous shape such as ones shown in Figure 11 may alternatively be utilized. Contemplated heads of the bulbous shapes are wider than their corresponding necks, facilitating an interlocking block pattern.

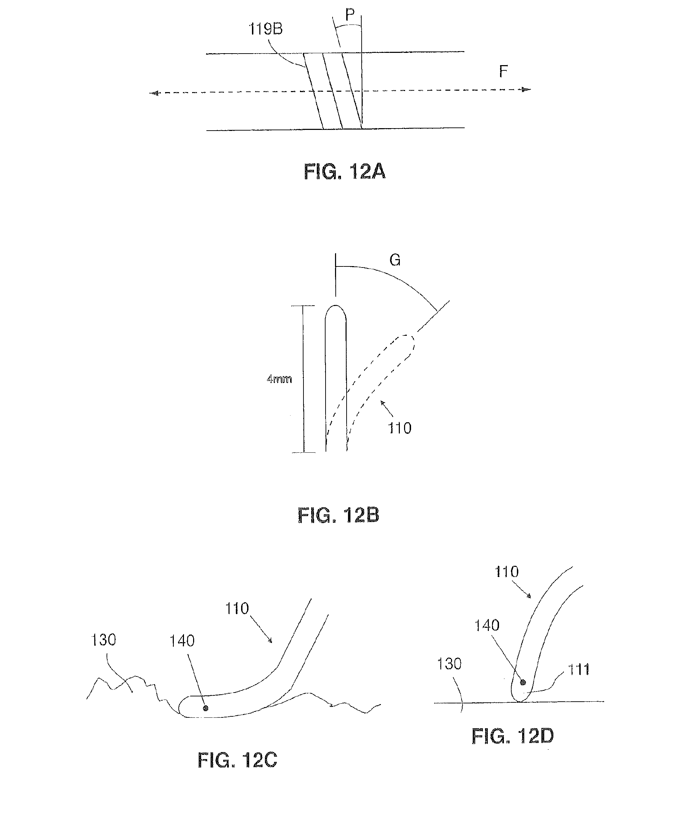

The stem members of Figures 10 and 11, for example, having an axis 119B, may extend in a helix about the longitudinal axis F in Figure 12A with a pitch P between and including 0.5 to 10 degrees. To describe it in another way, the patterns of elongated gaps 118 extend helically around the longitudinal axis F with a pitch angle, for example, between and including 0.5 to 10 degrees.

The contemplated elongated openings defining the gaps 118 between the blocks of the stem members (Figure 9) improve a flexibility of the electrode, and allow the electrode to flex and bend along the longitudinal length of the electrode and relative to the catheter body to which it is attached. For example, the ability of the electrode to flex allows an approximately 4 mm length of the electrode to bend at an angle G in Figure 12B that falls, for example, between and including 0.2 degrees to 70 degrees relative to the longitudinal axis from a substantially straight position. More specifically, the ability to flex allows the approximately 4 mm electrode length to bend between and including 5 degrees to 50 degrees relative to the longitudinal axis from a substantially straight position. Even more specifically, the ability to flex allows the approximately 4 mm length to bend about 45 degrees relative to the longitudinal axis from a substantially straight position.

Figures 12C and 12D illustrate a flexible electrode 110 being dragged across tissue 130. In Figure 12C, the electrode 110 is flexed and pressed against tissue 130, which has a relatively irregular surface. Being able to flex provides better contact with the target tissue, for example, in the trabeculated endocardial tissue where there are valleys, ridges, and pockets in the tissue surface. Here, electrode-to-tissue contact area is increased by using the side of the electrode 110 to deliver energy for ablation. The increased contact surface increases the likelihood of creating larger lesions at a given contact force and power setting. This in turn enables deeper ablation without having to increase the power setting, which is beneficial because increased power settings undesirably increase the likelihood of coagulation. In Figure 12D, the dome tip 111 is used to delivery energy to tissue 130.

The flexible electrode 110 also capably absorbs any contraction or vibration of tissue 130, and improves continuous tissue contact in a beating heart during systole and diastole, whether the electrode contacts the tissue 130 in a parallel, perpendicular, or other orientation. Continuous tissue contact is also assured regardless of whether the electrode is stationary at one location or when the electrode is in motion being dragged. Without such flexibility, a standard rigid tip electrode would "jump off' of the tissue in response to a beating heart.

Optionally, further embodiments of flexible electrodes for catheters may include force-sensing capability to measure contact force with body tissue in different directions. For example, a strain gage, a fiber optic sensor, or other sensor 140 (Figure 12C) may be disposed within the hollow electrode to measure an amount of force causing the electrode to flex, and to shorten as the case may be. Such data can be collected and communicated to the physician to monitor ablation progress. Monitoring of force experienced at the electrode may, for example, be used to prevent accidental piercing of the target tissue via too much perpendicular force being applied to press the dome 111 into the tissue.

Unlike known elongated electrodes (e.g., U.S. Patent No. 6,063,080), which can be laid across a tissue to create relatively longer linear lesions, the flexible electrodes as described have the unexpected advantage of improving precision in mapping and control at specific locations within the heart for more precise ablation, especially in relatively tight anatomical structures. Known elongated electrodes have difficulty positioning in such tight anatomical structures.

One unexpected advantage achieved with a flexible tip electrode is minimized "flipping." When a standard rigid tip electrode is manipulated within a body cavity having valleys and pockets in the tissue, the tip electrode can get caught or stuck in the tissue. As a physician continues to apply force in an attempt to move the tip electrode even though it is caught or stuck, the tip electrode may suddenly "flip" out of the tissue. Such "flipping" is highly undesirable and should be avoided. The proposed flexible tip electrodes greatly minimize "flipping" issues, and allow smoother dragging and motion across valleys and pockets in target tissue.

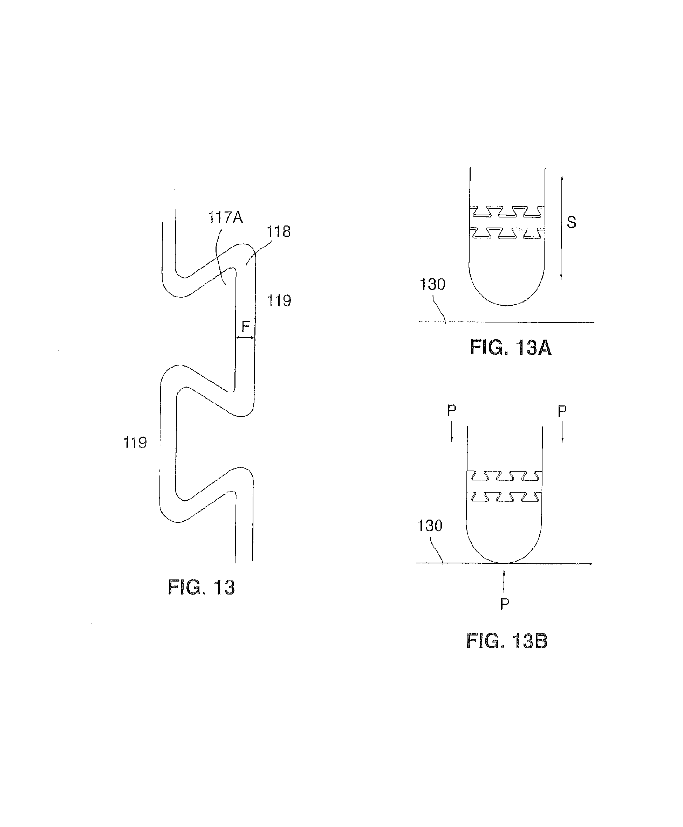

Referring now to Figure 13, the elongated openings in the wall provide a sufficient elongated gap 118 in the wall to allow shortening of a length of the electrode, when a force is applied to the electrode. The elongated gap 118 extends, for example, between a head 117A and a stem 119 of an adjacent loop in the electrode wall, and allows a freedom of movement F between adjacent stems along the longitudinal axis of the electrode wall when the elongated gap is narrowed or closed. Likewise, the elongated gap 118 between adjacent heads 117A provides a freedom of movement F for lengthening of the electrode along the longitudinal length of the electrode when the gap is opened or widened. Such shortening or lengthening may involve widening or narrowing one or more elongated gaps in the various embodiments described above.

In an exemplary embodiment, the electrode can shorten between and including 0.2% to 10% of an axial resting length of the electrode when the elongated gap(s) in the electrode wall are closed. In one embodiment the gap(s) in the electrode wall allows shortening of the axial length between and including 0.1% to 8% of the resting length. More specifically, the elongated gaps in the wall allow axial shortening of the length between and including 0.5% to 5% of the resting length, and even more specifically, the gaps in the wall allow shortening of the resting length between and including 0.1% to 0.5% of the length.

In Figure 13A, the electrode is at rest where no applied force is exerted thereon, and the electrode assumes a pre-determined shape stretching in the "S" direction and opening the elongated gap(s) to a predetermined amount. When the electrode contacts tissue 130 as shown in Figure 13B, an applied pressing force P causes the elongated gap(s) to narrow or close and the electrode to shorten, against the stretching force "S." Once shortened, the width of the elongated gap(s) providing freedom of movement F (Figure 13) in the direction of the applied force P may be minimized as depicted in Figure 13B. That is, the elongated gaps 118 may be fully closed such that the electrode length reaches a minimum axial length that is substantially unaffected by further exertion of applied force P.

In an exemplary embodiment, the stretching force "S" (Figure 13A) may be provided by a shape memory material or alloy used to fabricate the electrode wall. Alternatively, Figure 14A shows a cross sectional view of an electrode where the stretching force "S" is provided by a biasing element, such as a spring coil 122, in the lumen 120. The coil 122 provides structural integrity to the electrode wall and resiliently maintains the electrode in a pre-determined configuration in a resting state where no applied force is placed on the electrode. In one embodiment, the pre-determined electrode configuration at rest orients the longitudinal axis of the electrode to follow a straight line. In another embodiment, the pre-determined configuration at rest orients the longitudinal axis of the electrode along a curved or arcuate path as shown in Figure 14B. The contemplated coil resiliently biases the electrode to axially stretch in the direction of arrow S (Figure 14A) that is generally parallel to the longitudinal axis of the electrode. In other words, the coil optionally biases the tip electrode to stretch lengthwise. When deflected from the predetermined configuration under applied force, the electrode may resiliently return to the predetermined configuration when the applied force is released.

The coil 122, or the electrode, or both, may be fabricated from a shape memory material. The flexible tip electrode can be made of suitable conductive and biocompatible materials, suitable for ablation temperature; such materials include natural and synthetic polymers, various metals and metal alloys, Nitinol, naturally occurring materials, textile fibers, and combinations thereof. In one embodiment, the tip electrode is fabricated from MP3SN alloy.

Catheters having flexible tip electrodes such as those described above can be coupled to an irrigation system. That is, the catheter includes a fluid delivery lumen in the tubular catheter body, with the fluid delivery lumen in fluid communication with the hollow electrode. When one or more of the flexible tip electrodes change shape under an applied force, the elongated gap(s) will undergo changes in size and/or shape, thereby affecting the fluid flow therethrough. A cooling fluid, for example, may be pumped in an open flow path through the catheter body to the hollow lumen of the electrode, where it may pass through the gap(s) in the electrode wall to the exterior of the electrode, bathing the electrode and adjacent body tissue with cooling fluid. An internal, closed-loop irrigation system using re-circulated cooling fluid as known in the art is also possible. Also, catheters having flexible tip electrodes can be coupled to an energy source, such as a radio frequency (RF) generator to provide energy needed for tissue ablation. RF signal generators are known and are disclosed, for example, in U.S. Patent No. 6,235,022.

Flexible tip electrodes for ablation catheters may be formed and fabricated, for example, according to the following methodology. An exemplary method includes providing a hollow cylindrical electrode, and applying a laser to the cylindrical wall of the electrode to cut through a wall of the electrode. The laser cuts the wall in a pre-determined pattern that may extend helically around the circumference of the electrode wall, or may conform to any of the elongated groove or opening patterns previously described in the various embodiments above. As shown in Figure 15, the cuts may create an elongated gap 118 that may be consistently wider in some sections M and narrower in some other sections N. The wider sections M may extend substantially laterally from or transverse to a longitudinal axis (such as the axis F in Figure 12) of the electrode wall. The narrower sections N may connect the wider sections M together, and may be disposed generally in the direction of the longitudinal axis F of the electrode wall.

The wider sections M allow freedom of movement to narrow or widen the gap(s) 118 as previously described, making it possible to shorten an axial length of the electrode when a force is applied at a distal end of the electrode towards a proximal end.

Figure 15B illustrates still another embodiment where the laser also cuts the electrode wall in a predetermined pattern, where the elongated gap 118 created by the laser has generally consistent width. A coil may be subsequently installed in the lumen of this electrode to provide stretching force to create wider sections and narrower sections as previously described in relation to Figure 14A.

Coatings such as gold and platinum can be applied to the electrode to increase thermo-conductivity of the electrodes. The electrodes can also be coated with heparin to provide anticoagulation effect. In addition, the electrodes may be electro-polished to reduce sharp edges.

The many embodiments of flexible electrodes facilitate the following exemplary methods of performing linear ablation procedures. As with typical ablation catheters, a physician can perform mapping using the electrodes, and determine a target site for ablation. Once determined, the physician may drag the flexible tip electrode across the target tissue to start ablation while applying energy to the tissue. Because the electrode is flexible, the electrode can be more easily dragged across tissue surfaces having ridges and bumps while keeping constant electrode-to-tissue contact. And because the gaps in the electrode wall allows the electrode to be shortened when pressed tip-down against tissue surface, accidental tissue-piercing is largely avoided if not eliminated.

Thus, specific embodiments and applications of flexible tip electrodes have been disclosed. It should be apparent, however, to those skilled in the art that many more modifications besides those already described are possible without departing from the scope of the appended claims.

## Claims

1. An ablation catheter comprising
a catheter body; and
an electrode (110);
the electrode (110) comprising:
a hollow electrode body defined by a sidewall extending along a longitudinal axis (F); wherein
the sidewall is provided with a pattern including one or more elongated gaps (118) through a thickness of the wall, the elongated gaps (118) imparting flexibility to the sidewall to adopt different operating configurations relative to the longitudinal axis,
wherein the one or more elongated gaps comprise a gap that outlines alternating interlocking blocks (117),
wherein the electrode body further defines a fluid delivery lumen, the fluid delivery lumen in communication with the one or more elongated gaps (118),
wherein the catheter is configured to be coupled to an irrigation system such that when a cooling fluid is delivered in the lumen, it passes through the one or more elongated gaps (118) to the exterior of the electrode.

2. The catheter of claim 1, the electrode (110) further comprising a dome-shaped tip section (111) extending from the sidewall and forming a tip electrode.

3. The catheter of claim 1 or 2, wherein the different configurations include at least one of a resting length configuration, a shortened configuration, a substantially straight configuration, an arcuate configuration, and configurations having changed cross sectional shapes.

4. The catheter of any one of the preceding claims, wherein the sidewall is biased to a pre-determined configuration, preferably by the electrode (110) further comprising a coil (122) resiliently biasing the sidewall or by the electrode (110) being fabricated from a shape memory alloy positioning the electrode (110) in a predetermined configuration.

5. The catheter of any one of the preceding claims, wherein the sidewall is a substantially cylindrical sidewall provided with at least one elongated gap (118) formed in the wall, the at least one gap (118) extending as one or more of an annular gap around a portion of a circumference of the sidewall, a helical gap forming a helical pattern on the sidewall, a zig-zag gap forming a zig-zag pattern on the sidewall, and a wavy gap forming a wavy pattern on the sidewall.

6. The catheter of claim 1, wherein the one or more elongated gaps (118) are consistently wider in some sections and narrower in some sections,
wherein the wider sections are preferably substantially parallel to the longitudinal axis (F), and
wherein further preferably the one or more elongated gaps (118) spiral about the longitudinal axis (F) and the narrower sections are in between the wider sections, the narrower sections being transverse to the longitudinal axis (F) of the spiral.

7. The catheter of any one of the preceding claims, wherein the sidewall is bendable about 0.2 degrees to about 70 degrees relative to the longitudinal axis (F) from a substantially straight position or a predetermined configuration.

8. The catheter of any one of claims 1 to 6, wherein an approximately 4mm section of the electrode (110) is bendable about 5 degrees to about 50 degrees relative to the longitudinal axis (F) from a substantially straight position.

9. The catheter of any one of claims 1 to 6, wherein the sidewall is a substantially cylindrical sidewall provided with at least one elongated gap (118) to allow shortening of a length of the electrode between about 0.1% to about 10% of the length, preferably between about 0.2% to about 10% of a resting length of the electrode when a force is applied.

10. The catheter of any one of the preceding claims, wherein the sidewall comprises at least one stem (119) and opposing blocks (117) extending transversely from the stem (119).

11. The catheter of claim 10, wherein the opposing blocks (117) are disposed on opposite sides of the elongated gap (118), each block (117) having a head and a neck, and the head being wider than the neck, wherein preferably a first head, having a first neck connected to a first stem, is disposed between second and third heads, wherein said second head has a second neck and said third head has a third neck, wherein said second and third necks are connected to a second stem, and wherein further preferably the second and third heads has a shortest distance (A) between the two heads, and distance (A) is shorter than a widest width of the first head.

12. The catheter as recited in claim 11, wherein at least some of the blocks (117) have a shape of either an upside down triangle, or a bulbous shape.

13. The catheter of any one of the preceding claims, wherein the electrode (110) comprises a substantially cylindrical wall, the sidewall having at least one elongated gap (118) extending around the electrode gap with a pitch between about 0.5 to about 10 degrees.

## Patentansprüche

1. Ablationskatheter, enthaltend:
einen Katheterkörper; und
eine Elektrode (110);
wobei die Elektrode (110) enthält:
einen hohlen Elektrodenkörper, der durch eine Seitenwand definiert wird, die sich entlang einer Längsachse (F) erstreckt; wobei
die Seitenwand mit einem Muster versehen ist, das einen oder mehrere längliche Spalte (118) durch eine Dicke der Wand enthält, wobei die länglichen Spalte (118) der Seitenwand Flexibilität geben, dass sie unterschiedliche Betriebskonfigurationen in Bezug auf die Längsachse einnehmen kann,
wobei der eine oder die mehreren länglichen Spalte einen Spalt enthalten, der abwechselnde, miteinander arretierende Blöcke (117) abgrenzt,
wobei der Elektrodenkörper weiter ein Fluidzufuhrlumen definiert, wobei das Fluidzufuhrlumen in Verbindung mit dem einen oder mehreren länglichen Spalten (118) ist,
wobei der Katheter konfiguriert ist, mit einem Irrigationssystem gekoppelt zu werden, so dass, wenn ein Kühlfluid in dem Lumen zugeführt wird, es durch den einen oder die mehreren länglichen Spalte (118) zur Umgebung der Elektrode gelangt.

2. Katheter nach Anspruch 1, wobei die Elektrode (110) weiter einen kuppelförmigen Spitzenabschnitt (111) enthält, der sich von der Seitenwand aus erstreckt und eine Spitzenelektrode bildet.

3. Katheter nach Anspruch 1 oder 2, wobei die unterschiedlichen Konfigurationen zumindest eine der folgenden Konfigurationen enthalten: eine Ruhelängenkonfiguration, eine verkürzte Konfiguration, eine im Wesentlichen gerade Konfiguration, eine gebogene Konfiguration, und Konfigurationen, die veränderte Querschnittsformen aufweisen.

4. Katheter nach einem der vorhergehenden Ansprüche, wobei die Seitenwand in eine vorgegebene Konfiguration vorgespannt ist, bevorzugt durch die Elektrode (110), die weiter eine Spule (122) enthält, die die Seitenwand elastisch vorbelastet, oder durch die Elektrode (110), die aus einer Shape-Memory-Legierung hergestellt ist, die die Elektrode (110) in einer vorgegebenen Konfiguration positioniert.

5. Katheter nach einem der vorhergehenden Ansprüche, wobei die Seitenwand eine im Wesentlichen zylindrische Seitenwand ist, die mit zumindest einem länglichen Spalt (118) versehen ist, der in der Wand geformt ist, wobei der zumindest eine Spalt (118) sich erstreckt als einer oder mehrere aus einem ringförmigen Spalt um einen Bereich eines Umfangs der Seitenwand, einem helixförmigen Spalt, der ein Helixmuster auf der Seitenwand bildet, einem Zickzackspalt, der ein Zickzackmuster auf der Seitenwand bildet, und einem wellenförmigen Spalt, der ein Wellenmuster auf der Seitenwand bildet.

6. Katheter nach Anspruch 1, wobei der eine oder die mehreren länglichen Spalte (118) in einigen Abschnitten durchweg breiter und in einigen Abschnitten schmaler sind,
wobei die breiteren Abschnitte vorzugsweise im Wesentlichen parallel zur Längsachse (F) sind, und
wobei weiter vorzugsweise der eine oder die mehreren länglichen Spalte (118) sich spiralförmig um die Längsachse (F) winden und die engeren Abschnitte zwischen den breiteren Abschnitten sind, wobei die engeren Abschnitte quer zur Längsachse (F) der Spirale sind.

7. Katheter nach einem der vorhergehenden Ansprüche, wobei die Seitenwand um 0,2 Grad bis etwa 70 Grad relativ zur Längsachse (F) aus einer im Wesentlichen geraden Position oder einer vorgegebenen Konfiguration biegbar ist.

8. Katheter nach einem der Ansprüche 1 bis 6, wobei ein ungefähr 4mm-Abschnitt der Elektrode (110) um etwa 5 Grad bis etwa 50 Grad relativ zur Längsachse (F) aus einer im Wesentlichen geraden Position biegbar ist.

9. Katheter nach einem der Ansprüche 1 bis 6, wobei die Seitenwand eine im Wesentlichen zylindrische Seitenwand ist, die mit zumindest einem länglichen Spalt (118) versehen ist, dass eine Verkürzung einer Länge der Elektrode zwischen etwa 0,1% bis etwa 10% der Länge, vorzugsweise zwischen etwa 0,2% bis etwa 10% einer Ruhelänge der Elektrode ermöglicht ist, wenn eine Kraft aufgebracht wird.

10. Katheter nach einem der vorhergehenden Ansprüche, wobei die Seitenwand zumindest einen Stamm (119) und sich quer von dem Stamm (119) erstreckende, gegenüberliegende Blöcke (117) enthält.

11. Katheter nach Anspruch 10, wobei die gegenüberliegenden Blöcke (117) auf gegenüberliegenden Seiten des länglichen Spalts (118) angeordnet sind, wobei jeder Block (117) einen Kopf und einen Hals hat, und wobei der Kopf breiter als der Hals ist, wobei vorzugsweise ein erster Kopf, der einen ersten Hals mit einem ersten Stamm verbunden hat, zwischen einem zweiten und einem dritten Kopf angeordnet ist, wobei der zweite Kopf einen zweiten Hals und der dritte Kopf einen dritten Hals hat, wobei der zweite und der dritte Hals mit einem zweiten Stamm verbunden sind, und wobei weiter vorzugsweise der zweite und der dritte Kopf einen kürzesten Abstand (A) zwischen den zwei Köpfen aufweisen, und der Abstand (A) kürzer als eine größte Breite des ersten Kopfs ist.

12. Katheter nach Anspruch 11, wobei zumindest einige der Blöcke (117) eine Form von entweder einem umgekehrten Dreieck oder eine knollige Form haben.

13. Katheter nach einem der vorhergehenden Ansprüche, wobei die Elektrode (110) eine im Wesentlichen zylindrische Wand enthält, die Seitenwand zumindest einen länglichen Spalt (118) aufweist, der sich um den Elektrodenspalt mit einer Steigung von etwa 0,5 bis etwa 10 Grad erstreckt.

## Revendications

1. Un cathéter pour ablation comprenant :
un corps de cathéter; et
une électrode (110) ;
l'électrode (110) comprenant :
un corps d'électrode creux défini par une paroi s'étendant le long d'un axe longitudinal (F) ; dans lequel
la paroi est munie d'un motif incluant un ou plusieurs orifices allongés (118) à travers une épaisseur de la paroi, les orifices allongés (118) donnant de la flexibilité à la paroi pour adopter différentes configurations de fonctionnement par rapport à l'axe longitudinal,
dans lequel les un ou plusieurs orifices allongés comprennent un orifice qui délimite des blocs de verrouillage en alternance (117),
dans lequel le corps d'électrode définit en outre une lumière de distribution de fluide, la lumière de distribution de fluide en communication avec les un ou plusieurs orifices allongés (118),
dans lequel le cathéter est configuré pour être couplé à un système d'irrigation de sorte que quand un fluide de refroidissement est distribué dans la lumière, il passe à travers les un ou plusieurs orifices allongés (118) vers l'extérieur de l'électrode.

2. Le cathéter selon la revendication 1, l'électrode (110) comprenant en outre une section d'extrémité (111) en forme de dôme s'étendant depuis la paroi et formant une électrode d'extrémité.

3. Le cathéter selon la revendication 1 ou 2, dans lequel les différentes configurations incluent au moins une parmi une configuration de longueur restante, une configuration écourtée, une configuration substantiellement droite, une configuration arquée, et des configurations présentant des formes à section transversale modifiée.

4. Le cathéter selon l'une quelconque des revendications précédentes, dans lequel la paroi est contrainte à une configuration prédéterminée, de préférence par l'électrode (110) comprenant en outre une bobine (122) contraignant de manière résiliente la paroi ou par l'électrode (110) étant fabriquée à partir d'un alliage à mémoire de forme positionnant l'électrode (110) dans une configuration prédéterminée.

5. Le cathéter selon l'une quelconque des revendications précédentes, dans lequel la paroi est une paroi sensiblement cylindrique munie d'au moins un orifice allongé (118) formé dans la paroi, le au moins un orifice (118) s'étendant comme un ou plus parmi un orifice annulaire autour d'une portion d'une circonférence de la paroi, un orifice hélicoïdal formant un motif hélicoïdal sur la paroi, un orifice zigzag formant un motif en zigzag sur la paroi, et un orifice onduleux formant un motif onduleux sur la paroi.

6. Le cathéter selon la revendication 1, dans lequel les un ou plusieurs orifices allongés (118) sont considérablement plus larges dans certaines sections et plus étroits dans certaines sections,
dans lequel les sections plus larges sont avantageusement sensiblement parallèles à l'axe longitudinal (F), et
dans lequel en outre, avantageusement, les un ou plusieurs orifices allongés (118) tournent en spirale autour de l'axe longitudinal (F) et les sections plus étroites sont entre les sections plus larges, les sections plus étroites étant transversales à l'axe longitudinal (F) de la spirale.

7. Le cathéter selon l'une quelconque des revendications précédentes, dans lequel la paroi est pliable d'environ 0,2 degrés à environ 70 degrés par rapport à l'axe longitudinal (F) à partir d'une position sensiblement droite ou d'une configuration prédéterminée.

8. Le cathéter selon l'une quelconque des revendications 1 à 6, dans lequel une section de 4mm environ de l'électrode (110) est pliable d'environ 5 degrés à environ 50 degrés par rapport à l'axe longitudinal (F) à partir d'une position sensiblement droite.

9. Le cathéter selon l'une quelconque des revendications 1 à 6, dans lequel la paroi est une paroi sensiblement cylindrique munie d'au moins un orifice allongé (118) pour permettre le raccourcissement d'une longueur de l'électrode entre environ 0,1% et environ 10% de la longueur, de préférence entre environ 0,2% et environ 10% d'une longueur restante de l'électrode quand une force est appliquée.

10. Le cathéter selon l'une quelconque des revendications précédentes, dans lequel la paroi comprend au moins une tige (119) et des blocs opposés (117) s'étendant transversalement à partir de la tige (119).

11. Le cathéter selon la revendication 10, dans lequel les blocs opposés (117) sont disposés sur des côtés opposés de l'orifice allongé (118), chaque bloc (117) comprenant une tête et un collet, et la tête étant plus large que le collet, dans lequel de préférence une première tête, comprenant un premier collet connecté à une première tige, est disposée entre des seconde et troisième têtes, dans lequel ladite seconde tête comprend un second collet et ladite troisième tête comprend un troisième collet, dans lequel lesdits second et troisième collets sont connectés à une seconde tige, et dans lequel en outre, avantageusement, les seconde et troisième têtes présentent une distance la plus courte (A) entre les deux têtes, et la distance (A) est plus courte qu'une largeur la plus large de la première tête.

12. Le cathéter selon la revendication 11, dans lequel au moins certains des blocs (117) présentent soit une forme d'un triangle renversé, soit une forme bulbeuse.

13. Le cathéter selon l'une quelconque des revendications précédentes, dans lequel l'électrode (110) comprend une paroi sensiblement cylindrique, la paroi comprenant au moins un orifice allongé (118) s'étendant autour de l'orifice d'électrode avec un pas entre environ 0,5 et environ 10 degrés.
